(19) Europäisches Patentamt
European Patent Office
Office européen des brevets

(11) Veröffentlichungsnummer : **0 098 440**
**B1**

(12) # EUROPÄISCHE PATENTSCHRIFT

(45) Veröffentlichungstag der Patentschrift : **30.12.86**

(21) Anmeldenummer : **83105993.6**

(22) Anmeldetag : **20.06.83**

(51) Int. Cl.⁴ : **C 07 D215/18**, C 07 D401/12, C 07 D407/12, C 07 D409/12, C 07 D417/12, A 01 N 43/42, A 01 N 43/60, A 01 N 43/78

(54) **3,7-Dichlor-chinolinderivate, Verfahren zu ihrer Herstellung und ihre Verwendung zur Bekämpfung unerwünschten Pflanzenwuchses.**

(30) Priorität : **26.06.82 DE 3223884**

(43) Veröffentlichungstag der Anmeldung : **18.01.84 Patentblatt 84/03**

(45) Bekanntmachung des Hinweises auf die Patenterteilung : **30.12.86 Patentblatt 86/52**

(84) Benannte Vertragsstaaten : **CH DE FR GB IT LI**

(56) Entgegenhaltungen :
**EP-A- 0 060 429**
**FR-A- 2 183 265**
***Die Akte enthält technische Angaben, die nach dem Eingang der Anmeldung eingereicht wurden und die nicht in dieser Patentschrift enthalten sind.***

(73) Patentinhaber : **BASF Aktiengesellschaft**
**Carl-Bosch-Strasse 38**
**D-6700 Ludwigshafen (DE)**

(72) Erfinder : **Markert, Juergen, Dr.**
**Am Speyerweg 26**
**D-6704 Mutterstadt (DE)**
Erfinder : **Hagen, Helmut, Dr.**
**Max-Slevogt-Strasse 17e**
**D-6710 Frankenthal (DE)**
Erfinder : **Wuerzer, Bruno, Dr. Dipl.-Landwirt**
**Ruedigerstrasse 13**
**D-6701 Otterstadt (DE)**

**EP 0 098 440 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 3,7-Dichlor-chinolinderivate, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Chinolinderivate mit schwachen herbiziden Eigenschaften sind aus DE-OS 23 22 143 und US-PS 2 661 276 bekannt.

Es wurde gefunden, daß 3,7-Dichlor-chinolinderivate der Formel I

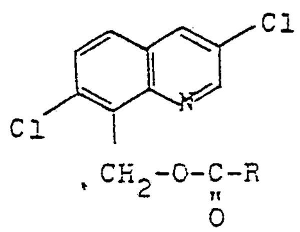

(I)

in der R für Wasserstoff, $C_1$-$C_8$-Alkyl, $C_2$-$C_6$-Alkenyl, $C_1$-$C_6$-Hydroxyalkyl, Cyclohexyl, für gegebenenfalls durch Nitro, Cyano, Benzoyl, Hydroxy, Methyl, Methoxy, Halogen, Trihalogenmethyl, Amino oder eine Dialkylaminogruppe mit 1 bis 4 C-Atomen in einer Alkylgruppe einfach oder mehrfach substituiertes Phenyl, für Pyridyl, Piperazinyl, Pyrazinyl, Piperidinyl, Thienyl, Furyl, Thiazolidinyl, Carboxyl, Trihalogenmethyl, für gegebenenfalls durch $C_1$-$C_4$-Alkyl substituiertes Benzyl, Benzophenonyl, Phenoxymethyl oder Anilinomethyl steht, eine beachtliche selektive herbizide Wirkung haben.

R in Formel I kann beispielsweise folgende Reste bedeuten : Wasserstoff, unverzweigtes oder verzweigtes $C_1$-$C_8$-Alkyl, wie Methyl, Ethyl, n-Propyl, n-Butyl, i-Butyl, n-Pentyl, n-Hexyl, n-Octyl, unverzweigtes oder verzweigtes $C_2$-$C_6$-Alkenyl, wie Allyl, Propenyl, unverzweigtes oder verzweigtes $C_1$-$C_6$-Hydroxyalkyl, wie Hydroxymethyl, 1-Hydroxyethyl, 2-Hydroxyethyl, 1-Hydroxy-isopropyl, 3-Hydroxy-n-propyl, 1-Hydroxy-3-methyl-n-butyl, gegebenenfalls durch Nitro, Cyano, Benzoyl, Hydroxy, Methyl, Methoxy, Halogen, wie Chlor, Fluor, Brom, Jod, Trihalogenmethyl, wie Trifluormethyl, Trichlormethyl, Amino oder eine Dialkylaminogruppe mit 1 bis 4 C-Atomen in einer Alkylgruppe, wie Dimethylamino, Diethylamino einfach oder mehrfach substituiertes Phenyl, Pyridyl, Piperazinyl, Pyrazinyl, Piperidinyl, Thienyl, Furyl, Thiazolidinyl, Carboxyl, Trihalogenmethyl, wie Trifluormethyl, Trichlormethyl, gegebenenfalls durch $C_1$-$C_4$-Alkyl, wie Methyl oder Ethyl, substituiertes Benzyl, Benzophenonyl, Phenoxymethyl oder Anilinomethyl. R in Formel I steht vorzugsweise für $C_1$-$C_8$-Alkyl, insbesondere für $C_1$-$C_4$-Alkyl, beispielsweise für Methyl.

Die Verbindungen der Formel I werden in an sich bekannter Weise durch Umsetzung von 3,7-Dichlor-8-chlormethyl-chinolin mit der ein- bis zweifachen molaren Menge eines Salzes einer Carbonsäure der Formel II

$$R-\underset{\underset{O}{\|}}{C}-O^{\ominus}\ M^{\oplus} \qquad (II)$$

in der R die obengenannten Bedeutungen hat und $M^{\oplus}$ für ein Alkalimetallion, wie Lithium, Natrium, Kalium, steht, erhalten.

Die Umsetzung wird in einem inerten Lösungsmittel, wie Dimethylformamid, Dimethylsulfoxid, einem Alkohol, beispielsweise Ethanol, Methylglykol, oder Wasser, durchgeführt. Sie ist nach 2 bis 6 Stunden beendet. Die Reaktionstemperatur liegt zwischen 80 und 100 °C.

Die Carbonsäuresalze der Formel II werden dabei entweder im jeweiligen Reaktionsmedium mit der berechneten Menge an Base, z. B. Natriummethylat, Kaliumethylat, Kaliumhydroxid, Natriumhydroxid, Natriumcarbonat, Natriumethylat, hergestellt oder als isolierte Salze zugegeben.

3,7-Dichlor-8-chlormethyl-chinolin kann aus 7-Chlor-8-methyl-chinolin durch Chlorierung in Dichlorbenzol bei 140 bis 160 °C hergestellt werden. Unter den geeigneten Bedingungen findet dabei neben der Seitenkettenchlorierung eine gleichzeitige selektive Kernchlorierung in 3-Stellung des Chinolins statt.

Beispiel 1

89 Gew.-Teile 7-Chlor-8-methylchinolin und 0,5 Gew.-Teile Azo-bis-isobutyronitril werden in 500 Gew.-Teilen Dichlorbenzol vorgelegt und auf 140 °C erhitzt. Bei dieser Temperatur wird mit dem Einleiten von 80 Gew.-Teilen Chlor begonnen. Während des Einleitens von Chlor wird die Temperatur auf 160 °C erhöht. Nach Beendigung der Chlorzugabe wird die Lösung mit Stickstoff gespült, der größte Teil des Lösungsmittels abdestilliert, der ausgefallene Feststoff abgesaugt und mit Petrolether gewaschen. Es

werden 113 Gew.-Teile 3,7-Dichlor-8-chlormethyl-chinolin vom Schmp. 129 °C erhalten. Die Ausbeute entspricht 93 % d. Th. 24,5 Gew.-Teile 3,7-Dichlor-8-chlormethyl-chinolin und 13 Gew.-Teile Natriumformiat werden in 220 Gew.-Teilen Dimethylsulfoxid 6 Stunden auf 100 °C erhitzt. Man kühlt ab, versetzt mit Wasser, saugt das ausgefallene Produkt ab und kristallisiert aus Toluol um. Man erhält 21,5 Gew.-Teile (84 % d. Th.) 3,7-Dichlor-8-formyl-oxymethyl-chinolin vom Schmp. 146 °C.

Beispiel 2

16 Gew.-Teile Nonancarbonsäure und 20 Gew.-Teile 30 %ige Natriummethylatlösung werden in 440 Gew.-Teile Dimethylsulfoxid 1 Stunde bei 100 °C gerührt. Anschließend werden 24,5 Gew.-Teile 3,7-Dichlor-8-chlormethyl-chinolin eingetragen, und das Reaktionsgemisch wird weitere 2 Stunden bei 100 °C gerührt. Nach dem Abkühlen wird die Reaktionslösung mit Wasser versetzt, der ausgefallene Feststoff wird abgesaugt und aus Toluol umkristallisiert. Es werden 32,5 Gew.-Teile (89 % d. Th.) 3,7-Dichlor-8-octylcarbonyl-oxymethyl-chinolin vom Schmp. 92 °C erhalten.

Beispiel 3

33 Gew.-Teile 2-Nitrobenzoesäure und 40 Gew.-Teile 30 %ige Natriummethylatlösung werden in 440 Gew.-Teilen Dimethylsulfoxid 2 Stunden bei 100 °C gerührt. Danach werden 49 Gew.-Teile 3,7-Dichlor-8-chlormethyl-chinolin eingetragen, und das Reaktionsgemisch wird weitere 2 Stunden bei 100 °C gerührt. Die Lösung wird abgekühlt, mit Wasser versetzt, der ausgefallene Feststoff wird abgesaugt und aus Toluol umkristallisiert. Man erhält 69 Gew.-Teile (92 % d. Th.) 3,7-Dichlor-8-[(2-nitrophenyl)-carbonyl-oxymethyl]-chinolin vom Schmp. 152 °C.

Analog können beispielsweise folgende Verbindungen der Formel I hergestellt werden :

(Siehe Tabelle Seite 4 f.)

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalin-derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanz-

| Verbindung Nummer | R | Schmp. (°C) |
|---|---|---|
| 4 | $CH_3$ | 144 |
| 5 | $C_2H_5$ | 140 |
| 6 | Phenyl | 184 |
| 7 | 4-Chlorphenyl | |
| 8 | Pyrid-3-yl | 177 |
| 9 | $CCl_3$ | |
| 10 | $CF_3$ | |
| 11 | Benzyl | 142 |
| 12 | Cyclohexyl | 156 |
| 13 | Phenoxymethyl | 126 |
| 14 | Anilinomethyl | 105 |
| 15 | Prop-1-enyl | 112 |
| 16 | 3,4,5-Trimethyl-phenyl | |
| 17 | tert.-$C_4H_9$ | 110 |
| 18 | Thien-3-yl | |
| 19 | Thien-2-yl | |
| 20 | n-$C_3H_7$ | |
| 21 | n-$C_4H_9$ | |
| 22 | n-$C_5H_{11}$ | |
| 23 | 2-Chlorphenyl | 165 |
| 24 | Piperid-4-yl | |
| 25 | 4-Methoxyphenyl | 197 |
| 26 | 2-Amino-phenyl | 169 |
| 27 | Fur-3-yl | |
| 28 | Fur-2-yl | |
| 29 | 4-Methylphenyl | 184 |
| 30 | 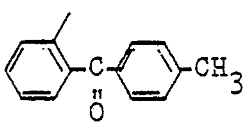 | 128 |
| 31 | Piperazin-2-yl | |
| 32 | 3,4,5-Trihydroxyphenyl | |
| 33 | Pyrid-4-yl | |
| 34 | Pyrid-2-yl | |

liche Produkte, wie Getreidemehl, Baumrinden-, Holz- und Nußschalenmehl, Cellulosepulver und andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind :

I. Man vermischt 90 Gewichtsteile des Wirkstoffs Nr. 1 mit 10 Gewichtsteilen N-Methyl-α-pyrrolidon und erhält eine Lösung, die zur Anwendung in Form kleinster Tropfen geeignet ist.

II. 20 Gewichtsteile des Wirkstoffs Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile des Wirkstoffs Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 4 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 8 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 4 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 17 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 13 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls vermischt. Man erhält eine stabile ölige Dispersion.

Die Applikation der Wirkstoffe bzw. der Mittel kann im Vorauflaufverfahren oder bei Nachauflaufanwendung erfolgen. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,1 bis 5 kg/ha und mehr, vorzugsweise 0,05 bis 4 kg/ha.

Die herbizide Wirkung von Verbindungen der Formel I wird durch Gewächshausversuche gezeigt :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach werden bei Vorauflaufanwendung die Wirkstoffe auf die Erdoberfläche aufgebracht. Sie werden hierzu in Wasser als Verteilungsmittel suspendiert oder emulgiert und mittels fein verteilender Düsen gespritzt. Die Aufwandmengen variieren dabei beispielsweise zwischen 2,0 und 4,0 kg Wirkstoff/ha. Nach dem Aufbringen der Wirkstoffe werden die Gefäße leicht beregnet, um Keimung und Wachstum in Gang zu bringen. Danach werden die Gefäße mit durchsichtigen Plastikhauben abgedeckt, bis die Pflanzen angewachsen sind. Diese Abdeckung bewirkt ein gleichmäßiges Keimen der Testpflanzen, sofern diese nicht durch die Wirkstoffe beeinträchtigt werden.

Für die Nachauflaufbehandlung werden die Testpflanzen je nach Wuchsform erst bis zu einer Wuchshöhe von 3 bis 15 cm angezogen und danach behandelt. Der für die Nachauflaufanwendung eingesetzte Reis wird in einem mit Torfmull (peat) angereicherten Substrat angezogen, um ein günstigeres Wachstum zu gewährleisten. Zur Nachauflaufbehandlung werden entweder direkt gesäte und in den gleichen Gefäßen aufgewachsene Pflanzen ausgewählt, oder aber sie werden erst als Keimpflanzen getrennt angezogen und einige Tage vor der Behandlung in die Versuchsgefäßte

verpflanzt. Eine Abdeckung unterbleibt bei der Nachauflaufbehandlung. Die Aufwandmenge für die Nachauflaufbehandlung beträgt beispielsweise 2,0 kg Wirkstoff/ha.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich bis zu 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die in den Gewächshausversuchen verwendeten Pflanzen setzten sich aus folgenden Arten zusammen :

Allium cepa (Küchenzwiebel), Amaranthus spp. (Fuchsschwanz), Beta vulgaris (Zuckerrübe), Brassica napus (Raps), Cassia tora, Euphorbia geniculata (Südamerik. Wolfsmilch), Echinochloa crus-galli (Hühnerhirse), Ipomoea spp. (Prunkwindearten), Galium aparine (Klettenlabkraut), Oryza sativa (Reis), Sesbania exaltata (Turibaum), Solanum nigrum (Schwarzer Nachtschatten), Triticum aestivum (Weizen).

In den Versuchen ist beispielsweise der Wirkstoff Nr. 4 bei Vor- und Nachauflaufanwendung von 2,0 kg/ha herbizid wirksam gegen unerwünschte breitblättrige und auch grasartige Pflanzen bei gleichzeitig guter Verträglichkeit für Kulturpflanzen, wie Raps, Reis, Küchenzwiebel und Weizen. Die Wirkstoffe Nr. 1 und 8 beispielsweise bekämpfen selektiv bei Vorauflaufanwendung von 2,0 bis 4,0 kg/ha unerwünschte Pflanzen in Raps bzw. Zuckerrüben. Ebenfalls im Vorauflaufverfahren zeigen die Wirkstoffe Nr. 2, 5, 13 und 17 bei einer Aufwandmenge von 2,0 kg/ha eine beachtliche Wirkung gegen unerwünschte Pflanzen, während sie in landwirtschaftlichen Kulturen, z. B. in Rüben und Raps, selektiv sind.

In Anbetracht der guten Verträglichkeit für zahlreiche breitblättrige und andere Kulturen und der Vielseitigkeit der Applikationsmethoden können die erfindungsgemäßen Herbizide oder diese enthaltende Mittel noch in einer weiteren großen Zahl von Kulturpflanzen zur Beseitigung unerwünschten Pflanzenwuchses eingesetzt werden.

In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum | |
| (Gossypium arboreum | |
| Gossypium herbaceum | Baumwolle |
| Gossypium vitifolium) | |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |

| Botanischer Name | Deutscher Name |
|---|---|
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche**

1. 3,7-Dichlorchinolinderivate der Formel I

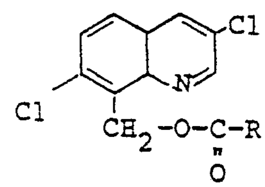

(I)

in der R für Wasserstoff, $C_1$-$C_8$-Alkyl, $C_2$-$C_6$-Alkenyl, $C_1$-$C_6$-Hydroxyalkyl, Cyclohexyl, für gegebenenfalls durch Nitro, Cyano, Benzoyl, Hydroxy, Methyl, Methoxy, Halogen, Trihalogenmethyl, Amino oder eine Dialkylaminogruppe mit 1 bis 4 C-Atomen in einer Alkylgruppe einfach oder mehrfach substituiertes Phenyl, für Pyridyl, Piperazinyl, Pyrazinyl, Piperidinyl, Thienyl, Furyl, Thiazolidinyl, Carboxyl, Trihalogenmethyl, für gegebenenfalls durch $C_1$-$C_4$-Alkyl substituiertes Benzyl, Benzophenonyl, Phenoxymethyl oder Anilinomethyl steht.

2. 3,7-Dichlorchinolinderivate der Formel I gemäß Anspruch 1, dadurch gekennzeichnet, daß R für $C_1$-$C_8$-Alkyl steht.

3. 3,7-Dichlor-8-methylcarbonyloxymethyl-chinolin.

4. Verfahren zur Herstellung von 3,7-Dichlorchinolin-derivaten der Formel I gemäß Anspruch 1, dadurch gekennzeichnet, daß man 3,7-Dichlor-8-chlormethyl-chinolin mit mindestens der molaren Menge eines Salzes einer Carbonsäure der Formel II

$$R - \underset{\underset{O}{\|}}{C} - O^{\ominus}\ M^{\oplus} \qquad (II)$$

in der R die im Anspruch 1 genannten Bedeutungen hat und $M^{\oplus}$ für ein Alkalimetallion steht, in einem inerten Lösungsmittel bei einer Temperatur zwischen 80 und 100 °C umsetzt.

5. Herbizid, enthaltend ein 3,7-Dichlorchinolinderivat der Formel I gemäß Anspruch 1.

6. Herbizid, enthaltend inerte Zusatzstoffe und ein 3,7-Dichlorchinolinderivat der Formel I gemäß Anspruch 1.

7. Herbizid, enthaltend inerte Zusatzstoffe und 0,1 bis 95 Gew.% eines 3,7-Dichlorchinolinderivats der Formel I gemäß Anspruch 1.

8. Herbizid, enthaltend inerte Zusatzstoffe und ein 3,7-Dichlorchinolinderivat der Formel I gemäß Anspruch 1, wobei R $C_1$-$C_8$-Alkyl bedeutet.

9. Herbizid, enthaltend inerte Zusatzstoffe und 3,7-Dichlor-8-methylcarbonyloxymethyl-chinolin.

10. Verfahren zur Bekämpfung unerwünschten Pflanzenwuchses, dadurch gekennzeichnet, daß man die Pflanzen oder ihren Standort mit einer herbizid wirksamen Menge eines 3,7-Dichlorchinolinderivats der Formel I gemäß Anspruch 1 behandelt.

**Claims**

1. A 3,7-dichloroquinoline derivative of the formula I

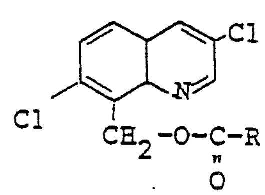

(I)

where R is hydrogen, $C_1$-$C_8$-alkyl, $C_2$-$C_6$-alkenyl, $C_1$-$C_6$-hydroxyalkyl, cyclohexyl, or phenyl which is unsubstituted or monosubstituted or polysubstituted by nitro, cyano, benzoyl, hydroxyl, methyl, methoxy, halogen, trihalomethyl, amino or dialkylamino, where alkyl is of 1 to 4 carbon atoms, or is pyridyl, piperazinyl, pyrazinyl, piperidinyl, thienyl, furyl, thiazolidinyl, carboxyl, trihalomethyl, or benzyl which is unsubstituted or substituted by $C_1$-$C_4$-alkyl, or is benzophenonyl, phenoxymethyl or anilinomethyl.

2. A 3,7-dichloroquinoline derivative of the formula I as claimed in claim 1, where R is $C_1$-$C_8$-alkyl.

3. 3,7-Dichloro-8-methylcarbonyloxymethylquinoline.

4. A process for the preparation of a 3,7-dichloroquinoline derivative of the formula I as claimed in claim 1, wherein 3,7-dichloro-8-chloromethylquinoline is reacted with at least an equimolar amount of a carboxylate of the formula II

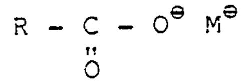

(II)

where R has the meanings given in claim 1, and $M^{\oplus}$ is an alkali metal ion, in an inert solvent at a temperature of from 80 to 100 °C.

5. A herbicide containing a 3,7-dichloroquinoline derivative of the formula I as claimed in claim 1.

6. A herbicide containing inert additives and a 3,7-dichloroquinoline derivative of the formula I as claimed in claim 1.

7. A herbicide containing inert additives and from 0.1 to 95 wt% of a 3,7-dichloroquinoline derivative of the formula I as claimed in claim 1.

8. A herbicide containing inert additives and a 3,7-dichloroquinoline derivative of the formula I as claimed in claim 1, R denoting $C_1$-$C_8$-alkyl.

9. A herbicide containing inert additives and 3,7-dichloro-8-methylcarbonyloxymethylquinoline.

10. A process for combating the growth of unwanted plants, wherein the plants or their location are treated with a herbicidally effective amount of a 3,7-dichloroquinoline derivative of the formula I as claimed in claim 1.

**Revendications**

1. Dérivés de 3,7-dichloroquinoline de formule I

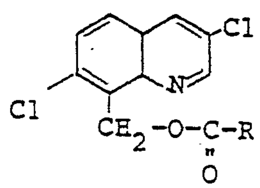

(I)

dans laquelle R représente hydrogène, alkyle en $C_1$-$C_8$, alcényle en $C_2$-$C_6$, hydroxyalkyle en $C_1$-$C_6$, cyclohexyle, phényle, éventuellement substitué une fois ou plusieurs fois par nitro, cyano, benzoyle, hydroxy, méthyle, méthoxy, halogène, trihalogénométhyle, amino ou un groupe dialkylamino de 1 à 4 atomes-C dans un groupe alkyle, ou représente encore pyridyle, pipérazinyle, pyrazinyle, pipéridinyle, thiényle, furyle, thiazolidinyle, carboxyle, trihalogénométhyle, ou représente encore benzyle, éventuellement substitué par alkyle en $C_1$-$C_4$, ou encore benzophénonyle, phénoxyméthyle ou anilinométhyle.

2. Dérivés de 3,7-dichloroquinoline de formule I selon la revendication 1, caractérisés par le fait que R représente alkyle en $C_1$-$C_8$.

3. 3,7-dichloro-8-méthylcarbonyloxyméthyl-quinoline.

4. Procédé de préparation de dérivés de 3,7-dichloroquinoline de formule I selon la revendication 1, caractérisé par le fait que l'on fait réagir, dans un solvant inerte, à une température comprise entre 80 et 100 °C, de la 3,7-dichloro-8-chlorométhylquinoline avec au moins la quantité molaire d'un sel d'acide carboxylique de formule II

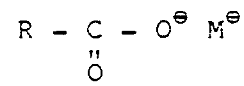

(II)

dans laquelle R a la signification indiquée dans la revendication 1 et $M^{\oplus}$ est mis pour un ion métal alcalin.

5. Herbicide, contenant un dérivé de 3,7-dichloroquinoline de formule I selon la revendication 1.

6. Herbicide, contenant des additifs inertes et un dérivé de 3,7-dichloroquinoline de formule I selon la revendication 1.

7. Herbicide, contenant des additifs inertes et 0,1 à 95 % en poids d'un dérivé de 3,7-dichloroquinoline de formule 1 selon la revendication 1.

8. Herbicide, contenant des additifs inertes et un dérivé de 3,7-dichloroquinoline de formule I selon la revendication 1, où R représente alkyle en $C_1$-$C_8$.

9. Herbicide, contenant des additifs inertes et une 3,7-dichloro-8-méthylcarbonyloxyméthyl-quinoline.

10. Procédé de lutte contre la croissance de plantes indésirables, caractérisé par le fait que l'on traite les plantes ou leur biotope avec une quantité efficace au point de vue herbicide d'un dérivé de 3,7-dichloroquinoline de formule I selon la revendication 1.